# EUROPEAN PATENT APPLICATION

(11) **EP 1 541 184 A1**
(43) Date of publication of application: **15.06.2005**
(21) Application number: 03784531.0
(22) Date of filing: 06.08.2003
(51) Int. Cl.: A61M 5/142

(54) **MEDICINE LIQUID INJECTION DEVICE FOR INJECTING PLURAL KINDS OF MEDICINE LIQUID WITHOUT MIXING THEM**

(30) Priority: 08.08.2002 JP 2002231159
(71) Applicant: Nemoto Kyorindo Co., Ltd., Tokyo 113-0033 (JP)
(72) Inventor: NEMOTO, Shigeru, c/o Nemoto Kyorindo Co., Ltd., Bunkyo-ku, Tokyo 113-0033 (JP)
(74) Representative: Burbaud, Eric
(86) International application number: PCT/JP2003/009971
(87) International publication number: WO 2004/014465

(57) **Abstract**

The present invention provides liquid injector 100 in which a patient is connected to a plurality of syringes 201, 202 trough patient tube 223 and a plurality of syringe tube 221, 222. When a liquid is injected into the patient from one of syringes 201, 202, syringe tube 222 or 221 connected to the other of syringes 202, 201 is blocked, so that the liquid injected into the patient from the one of syringes 201, 202 does not flow back into the other of syringes 202, 201. Therefore, it is possible to provide liquid injector 100 capable of preventing a backflow of a plurality of kinds of liquids freely injected into a patient.

## Description

### Technical Field

The present invention relates to a liquid injector for injecting a liquid into a patient, and more particularly, to a liquid injector for injecting liquids from a plurality of syringes into a patient.

### Background Art

Currently, CT (Computed Tomography) scanners used in medical facilities can create cross-sectional images of a patient by the application of X-ray imaging. MRI (Magnetic Resonance Imaging) apparatuses can create cross-sectional images of a patient in real time with the magnetic resonance effect. Angiographic apparatuses can image blood vessels of a patient by the application of the X-ray imaging.

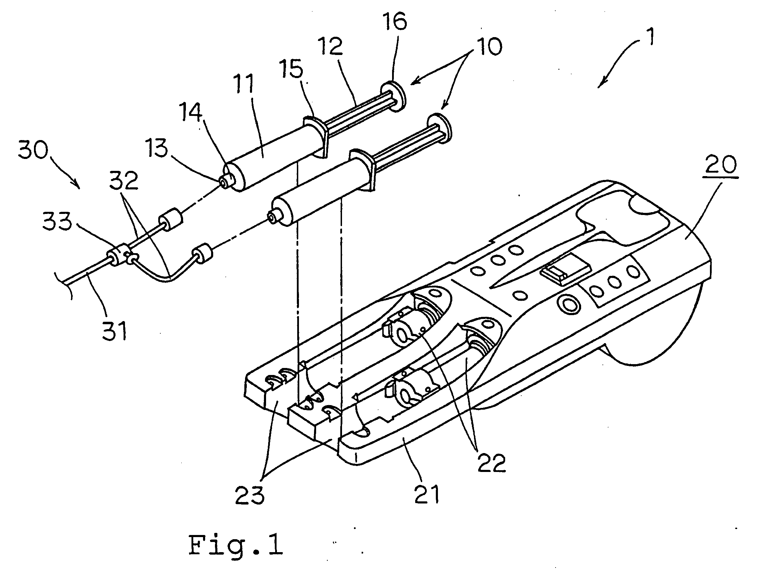

When the abovementioned apparatuses are used, a patient may be injected with a liquid such as a contrast medium and saline. Liquid injectors for automatically performing the injection have been put into practical use. A conventional example of such liquid injectors is hereinafter described with reference to Fig. 1.

Liquid injector 1 illustrated herein includes a number of replaceable syringes 10 and one liquid injector section 20. Two syringes 10, at most, are set to liquid injector section 20. Each of syringes 10 consists of one cylinder member 11 and one piston member 12. Cylinder member 12 has one hole portion 13 formed therein to open at its trailing end face.

Cylinder member 11 is closed at its leading end face and has hollow conduit portion 14 formed therein in the center. Hole portion 13 extends along conduit portion 14 to the leading end of conduit portion 14. Piston member 12 is slidably inserted into hole portion 13 of cylinder member 11. Piston flange 15 and cylinder flange 16 are formed on the circumference at the trailing ends of cylinder member 11 and piston member 12, respectively.

Liquid injector section 20 has one syringe hold member 21 and two syringe drive mechanisms 22. Two concaves 23 for individually holding cylinder members 11 of two syringes 10 are formed in one syringe hold member 21. Two syringe drive members 22 are individually disposed in the rear of two concaves 23. Syringe drive mechanisms 22 hold and slid piston members 12 of syringes 10.

Liquid injector 1 also has branch tube 30 which includes tube connecting member 33 serving as one tube connecting means for connecting the trailing end of one patient tube 31 to the leading ends of two syringe tubes 32. The trailing ends of two syringe tubes 32 of branch tube 30 are individually connected to conduits 14 of two syringes 10. A catheter, for example, is connected through an extension tube to the leading end of patient tube 31 of branch tube 30, and the catheter is connected to a patient (not shown).

With the structure as described above, liquid injector 1 of the conventional example allows two kinds of liquids to be injected into a patient from two syringes 10 with one liquid injector section 20. For example, it is possible that a contrast medium and then saline are injected into a patient whose cross-sectional images are to be created with a CT scanner.

In liquid injector 1 described above, however, the patient is simply connected to two syringes 10 through branch tube 30. When one of two syringes 10 is driven to inject a liquid into the patient, that liquid may flow back into the other of syringes 10. To prevent such a backflow, some measures may be taken including a switching valve contained in tube connecting member 33 of branch tube 30, or a one-way valve inserted into syringe tube 32 of branch tube 30 (neither shown).

When the switching valve is contained, however, the manual operation thereof is troublesome to possibly cause erroneous operation of driving syringe 10 connected to closed syringe tube 32. In liquid injector 1, a small amount of blood may be sucked from a patient with syringe 10 in order to ensure that patient tube 31 is appropriately connected to the patient. This operation cannot be performed in the liquid injector of the structure in which the one-way valve is inserted into syringe tube 32.

### Disclosure of Invention

The present invention has been made in view of the abovementioned problems, and it is an object of the present invention to provide a liquid injector capable of easily injecting a plurality of kinds of liquids to a patient and preventing a backflow of the liquid.

A liquid injector according to the present invention injects a liquid into a patient from a syringe having a cylinder member and a piston member inserted slidably into the cylinder member. The liquid injector has a patient tube, a plurality of syringe tubes, a tube connecting means, a syringe hold member, a plurality of syringe drive mechanisms, a plurality of tube block mechanisms, and an interlock control means.

The patient tube has a leading end connected to the patient. The plurality of syringe tubes are connected to the plurality of syringes, individually. The tube connecting means connects a trailing end of the patient tube to leading ends of the plurality of syringe tubes. The syringe hold member removably holds the plurality of syringes. Each of the plurality of syringe drive mechanisms causes each of the plurality of syringes to perform injection of the liquid by relatively moving the cylinder member and/or the piston member. The plurality of tube block mechanisms individually block the plurality of syringe tubes to allow opening or closing thereof. The interlock control means interlocks the operation of the plurality of tube block mechanisms and the plurality of syringe drive mechanisms to match the syringes tubes, only one of which is connected, with the syringes, only one of which is driven.

Therefore, in the liquid injector according to the present invention, the patient is connected to the plurality of syringes through the patient tube and the plurality of syringe tubes, but when the liquid is injected into the patient from one of the plurality of syringes, the syringe tube connected to the other syringe is blocked. It is thus possible to prevent the liquid injected from the one syringe into the patient from flowing back into the other syringe.

Various means referred to in the present invention may be any as long as it is formed to realize the function, and for example, can be realized as dedicated hardware which performs a predetermined function, a data processing apparatus which has a predetermined function provided by a computer program, a predetermined function realized inside a data processing apparatus by a computer program, a combination thereof, and the like.

Various components referred to in the present invention do not need to be independent items, and it is possible that a plurality of components is formed as one member, a component is contained as part of another component, and a component shares a portion with another component.

### Brief Description of the Drawings

Fig. 1 is a perspective view showing the outer appearance of a liquid injector of a conventional example.
Fig. 2 is a perspective view showing the outer appearance of major portions of a liquid injector of an embodiment according to the present invention.
Fig. 3 is a perspective view showing the outer appearance of the whole liquid injector.
Fig. 4 is a perspective view showing the outer appearances of the liquid injector and a CT scanner.
Fig. 5 is a block diagram showing the circuit configuration of the liquid injector.
Figs. 6a and 6b are schematic plan views showing the operation of the main portions of the liquid injector.
Fig. 7 is a flow chart showing a method of injecting liquids with the liquid injector.
Fig. 8 is a plan view showing the main portions of a first variation.
Fig. 9 is a plan view showing the main portions of a second variation.
Figs. 10a and 10b are perspective views showing the main portions of third variation.
Fig. 11 is a rear view showing the main portions of the third variation.

### Best Mode for Carrying Out the Invention

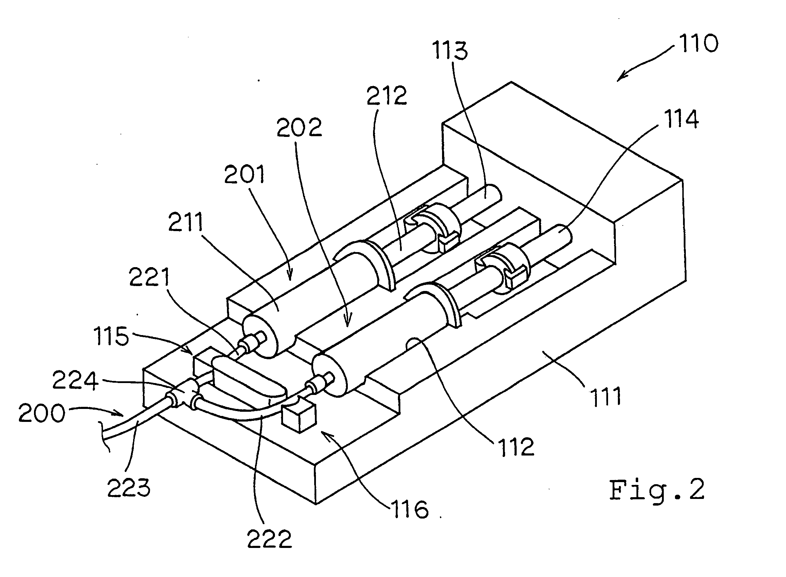

An embodiment of the present invention is hereinafter described with reference to Figs. 2 to 6. In the embodiment, components identical to those in the abovementioned conventional example are designated with the same names and detailed description thereof is omitted.

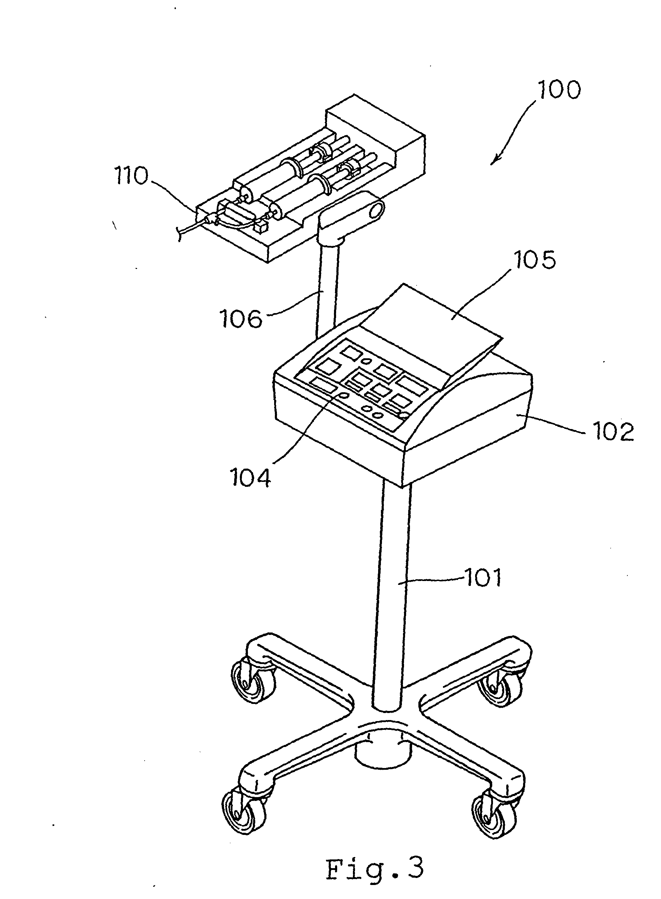

As shown in Fig. 3, liquid injector 100 of the embodiment has body 102 set on the top end of stand 101. Operation panel 104 and liquid crystal display 105 are mounted on body 102. Arm 106 is attached to the side of body 102. Injection head 110 serving as a syringe hold member is attached to the top end of arm 106.

As shown in Fig. 2, injection head 110 has two concaves 112 on the top surface of syringe hold member 111. Cylinder members 211 of first and second syringes 201, 202 are individually and removably held in concaves 112. First syringe 201 contains a contrast medium with high viscosity as a liquid, and second syringe 202 contains saline with low viscosity as a liquid (neither shown).

First and second drive mechanisms 113, 114 are contained in a rear portion of injection head 110 to serve as a first syringe drive mechanism and a second syringe drive mechanism, respectively. First and second drive mechanisms 113, 114 individually hold and slide piston members 212 of first and second syringes 201, 202.

Similarly to conventional liquid injector 1, in liquid injector 100, branch tube 220 has first and second tubes 221, 222 serving as a first syringe tube and a second syringe tube, respectively, connected to patient tube 223 through tube connecting member 224 serving as a tube connecting means. Branch tube 220 is used to connect first and second syringes 201, 202 to a patient (not shown).

First and second block mechanisms 115, 116 serving as a first tube block mechanism and a second tube block mechanism, respectively, are provided in the front of injection head 110. Each of first and second block mechanisms 115, 116 blocks each of first and second tubes 221, 222 to allow opening or closing thereof.

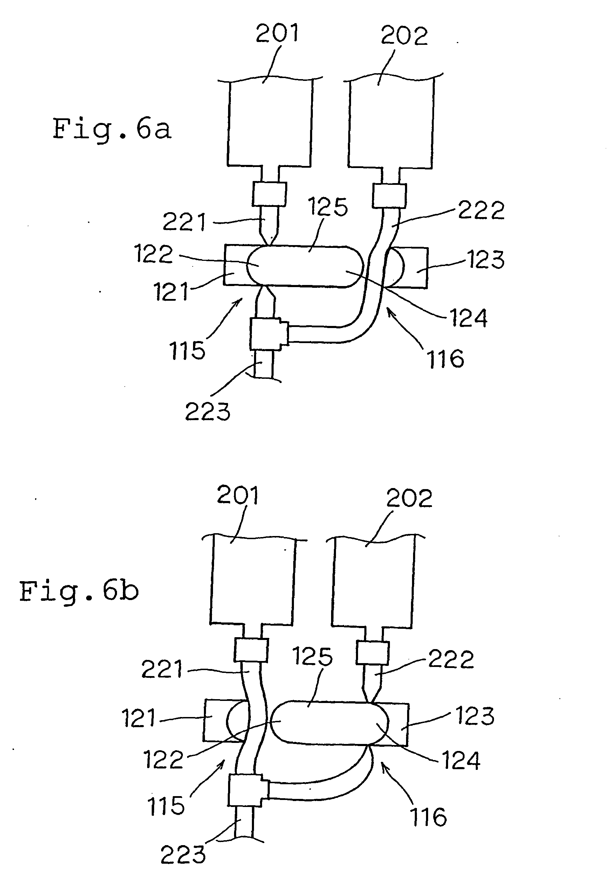

More specifically, as shown in Figs. 6a and 6b, first block mechanism 115 has first hold member 121 and first press member 122, while second block mechanism 116 has second hold member 123 and second press member 124. First hold member 121 is disposed at a position opposite to first press member 122 through first tube 221, while second hold member 123 is disposed at a position opposite to second press member 124 through second tube 222.

First press member 122 and second press member 124 are formed to be integral with both ends of one press slider member 125 on the left and the right, and press slider member 125 is supported to be slidable to left and right by an open or close interlock mechanism (not shown). Thus, first and second block mechanisms 115, 116 are interlocked such that one of them is opened when the other is closed.

The portions of first and second press members 122, 124 opposite to first and second hold members 121, 123 are formed on convex surfaces of a cylinder. The portions of first and second hold members 121, 123 opposite to first and second press members 122, 124 are formed on concave surfaces of the cylinder.

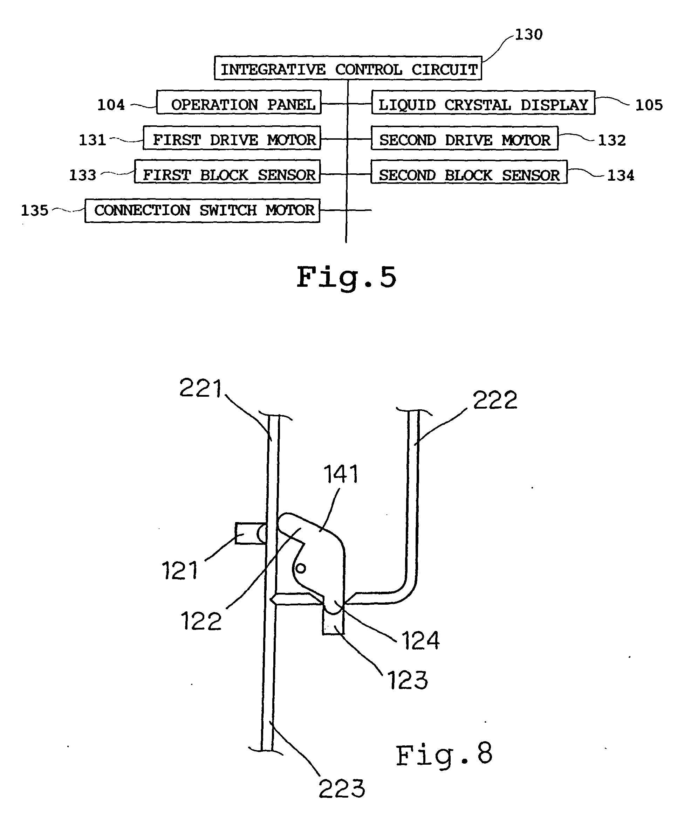

As shown in Fig. 5, liquid injector 100 of the embodiment has integrative control circuit 130 serving as an interlock control means. Operation panel 104, liquid crystal display 105, first drive motor 131, second drive motor 132, first block sensor 133, second block sensor 134, connection switch motor 135 and the like are connected to integrative control circuit 130.

First and second drive motors 131, 132 are drive sources for individually operating first and second drive mechanisms 113, 114. Connection switch motor 135 is a drive source for operating the open or close interlock mechanism. Since connection switch motor 135 is connected to press slider member 125 of the open or close interlock mechanism, for example through a worm gear (not shown), press slider member 125 is fixedly held at a position to which it is slid. First and second block sensors 133, 134 are realized, for example by photosensors (not shown) for detecting the position of press slider member 125, to individually sense the closing of first and second block mechanisms 115, 116.

Integrative control circuit 130 is realized by a so-called microcomputer and integrally controls the abovementioned sections in accordance with implemented computer programs. Although details are described later, integrative control circuit 130 thus interlocks the operation of first and second block mechanisms 115, 116 and first and second drive mechanisms 113, 114 to match first and second tubes 221, 222, only one of which is connected, with first and second syringes 201, 202, only one of which is driven.

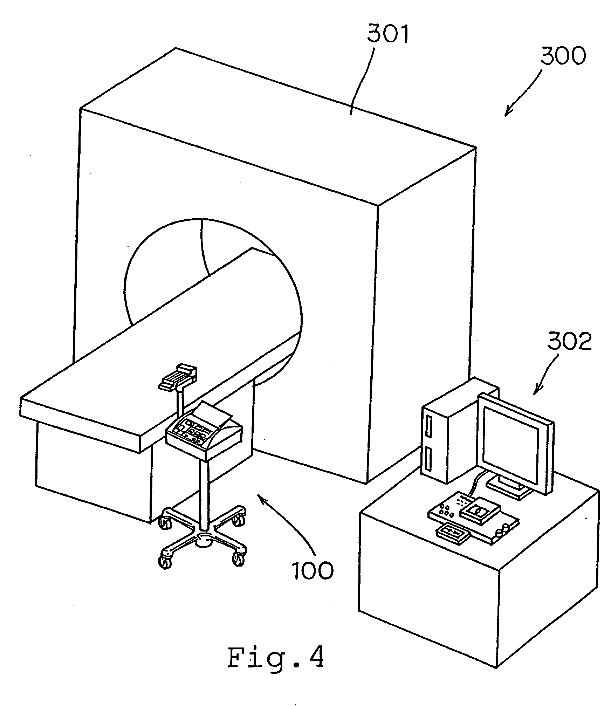

As shown in Fig. 4, liquid injector 100 of the embodiment is used near imaging unit 301 of CT scanner 300 and is connected to control unit 302 of CT scanner 300 as required. Control unit 302 is realized by a computer system and controls the operation of imaging unit 301 and displays cross-sectional images.

### Operation Of The Embodiment

With the structure as described above, liquid injector 100 of the embodiment can freely inject a contrast medium and saline, which are liquids, into a patient who is subjected to imaging with CT scanner 300, by way of example. In this case, an operator connects patient tube 223 of branch tube 220 to the patient and connects first and second tubes 221, 222 of branch tube 220 to first and second syringes 201, 202, respectively.

The operator then sets first and second syringes 201, 202 into concaves 112 of injection head 110 of liquid injector 100 and passes first and second tubes 221, 222 through first and second block mechanisms 115, 116 of injection head 110.

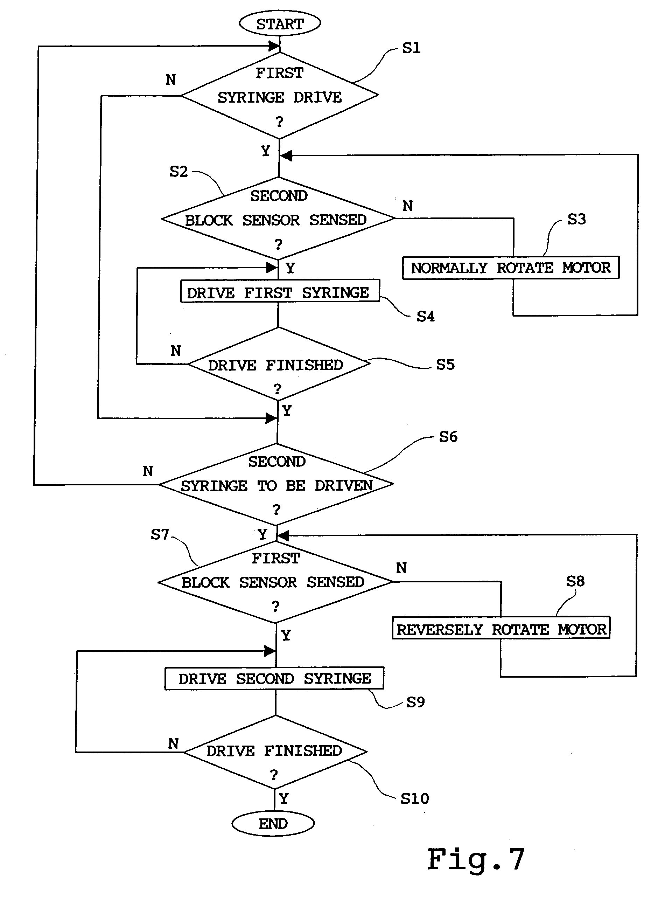

In this state, for example, when the contrast medium is injected to the patient from first syringe 201, the operator performs predetermined operation on operation panel 104 of liquid injector 100. Then, as shown in Fig. 7, integrative control circuit 130 senses the operation (step S1) and normally rotates connection switch motor 135 until second block sensor 134 senses the closing of second block mechanism 116 (steps S2 and S3).

In this state, integrative control circuit 130 activates first drive motor 131 for first drive mechanism 113 to inject the contrast medium from first syringe 201 into the patient (step S4). Integrative control circuit 130 stops first drive mechanism 113 to terminate the injection of the contrast medium in response to the operator making input to operation panel 104 for stopping the injection or in response to first drive mechanism 113 operating to a set position (step S5).

Similarly, when the saline is injected to the patient from second syringe 202, integrative control circuit 130 senses predetermined operation on operation panel 104 (step S6) and reversely rotates connection switch motor 135 until first block sensor 133 senses the closing of first block mechanism 115 (steps S7 and S8). Integrative control circuit 130 then activates second drive mechanism 114 to inject the saline into the patient from second syringe 202 (steps S9 and S10).

### Effect Of The Embodiment

In liquid injector 100 of the embodiment, the patient is connected to first and second syringes 201, 202 through patient tube 223 and first and second tubes 221, 222 as described above. It is thus possible to freely inject the contrast medium and the saline into the patient from first and second syringes 201, 202.

In addition, second tube 222 connected to second syringe 202 is blocked as shown in Fig. 6(b) when the contrast medium is injected into the patient from first syringe 201. This prevents a backflow of the contrast medium in first syringe 201 into second syringe 202.

Similarly, when the saline is injected into the patient from second syringe 202, first tube 221 connected to first syringe 201 is blocked as shown in Fig. 6(a) and therefore the saline in second syringe 202 does not flow back into first syringe 201.

Particularly, since first and second press members 122, 124 and first and second hold members 121, 123 block first and second tubes 221, 222 with the convexes and the concaves of the cylinder, a backflow of the contrast medium or the saline can be prevented reliably.

In addition, liquid injector 100 of the embodiment does not employ a one-way valve for preventing a backflow as described above. This allows, for example, a small amount of blood to be sucked from the patient with second syringe 202 in order to ensure that patient tube 223 is appropriately connected to the patient. In this case, first tube 221 which is not used to suck blood is blocked, so that the contrast medium in first syringe 201 can be prevented from being sucked into second syringe 202.

Furthermore, in liquid injector 100 of the embodiment, the drive of first and second syringes 201, 202 and the opening or closing of first and second tubes 221, 222 are automatically controlled in association as described above. Thus, liquid injector 100 does not suffer erroneous operation, for example driving first syringe 201 while first tube 221 is blocked, and a complicated action is not necessary such as manual operation of a switching valve.

Moreover, in liquid injector 100 of the embodiment, first and second block mechanisms 115, 116 are formed as the integral unit, and second block mechanism 116 reliably opens second tube 222 when first block mechanism 115 blocks first tube 221, and first block mechanism 115 reliably opens first tube 221 when second block mechanism 116 blocks second tube 222. Consequently, one of first and second tubes 221, 222 can be selectively blocked without fail.

### Variations Of The Embodiment

The present invention is not in any way limited to the abovementioned embodiment, and a number of variations are permitted without departing from the spirit and scope thereof. For example, while the abovementioned embodiment has shown liquid injector 100 which has two syringes 201, 202 mounted thereon, a liquid injector (not shown) which has three or more syringes mounted thereon can be realized.

Also, while first and second tubes 221, 222 are connected to patient tube 223 through separate tube connecting member 224 in the abovementioned embodiment, it is possible that first and second tubes 221, 222 and patient tube 223 are formed as an integral part.

In addition, while the abovementioned embodiment has shown that none of first and second tubes 221, 222 and patient tube 223 have a one-way valve, at least one of them can contain a one-way valve. However, when a small amount of blood is sucked from the patient with second syringe 202 in order to ensure that patient tube 223 is appropriately connected to the patient as described above, such a one-way valve is preferably contained only in first tube 221.

While first and second block mechanisms 115, 116 are formed as the integral unit and one of first and second tubes 221, 222 is selectively blocked in the abovementioned embodiment, the first and second block mechanisms can be formed separately and operated individually.

In this case, one of first and second tubes 221, 222 can be selectively opened and the other can be closed as described above. However, it is preferable that both of first and second tubes 221, 222 are normally blocked, and only when one of first and second syringes 201, 202 is driven, the associated one of first and second tubes 221, 222 is opened.

The abovementioned embodiment has shown that press slider member 125 of the open or close interlock mechanism is connected to connection switch motor 135 through the worm gear and thus press slider member 125 is fixedly held when connection switch motor 135 is stopped. Alternatively, connection switch motor 135 can be realized by a stepping motor to hold press slider member 125, or a dedicated lock mechanism (not shown) can be additionally provided to hold press slider member 125.

While the abovementioned embodiment has shown that the portions of first and second press members 122, 124 and first and second hold members 121, 123 for blocking first and second tubes 221, 222 are formed of the convexes of the cylinder and the concaves fit thereto, the portions can be planes or S-shaped portions.

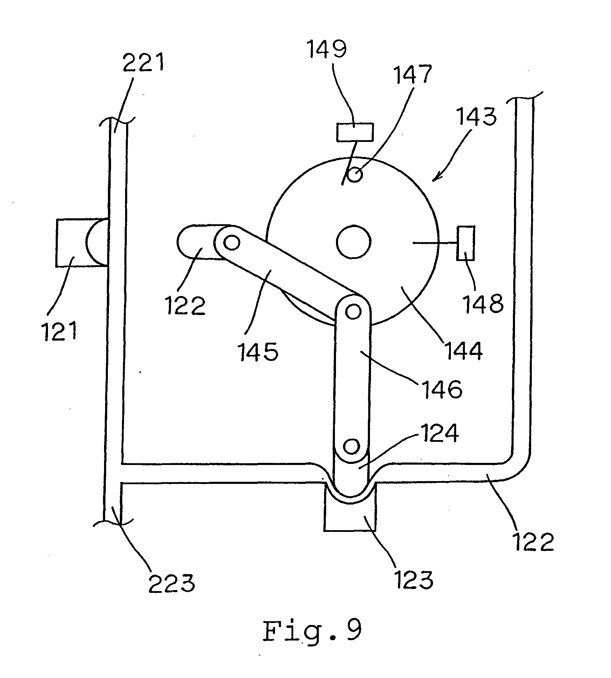

In the abovementioned embodiment, the open or close interlock mechanism selectively blocks one of first and second tubes 221, 222 with slidably supported press slider member 125. As an alternative example, as shown in Fig. 8, pivotally supported press pivot member 141 can be used to selectively block one of first and second tubes 221, 222.

Alternatively, as shown in Fig. 9, first press member 122 and second press member 124 can be slidably supported by a guide rail (not shown) and the like, and connected to crank member 144 of open or close interlock mechanism 143 through first link member 145 and second link member 146, respectively.

In open or close interlock mechanism 143, crank member 144 is formed in discoid shape, and convex 147 formed on the surface abuts on first and second block sensors 148, 149 which are realized by mechanical switches. It is thus possible to reliably sense the selective blocking of one of first and second tubes 221, 222 with the simple structure.

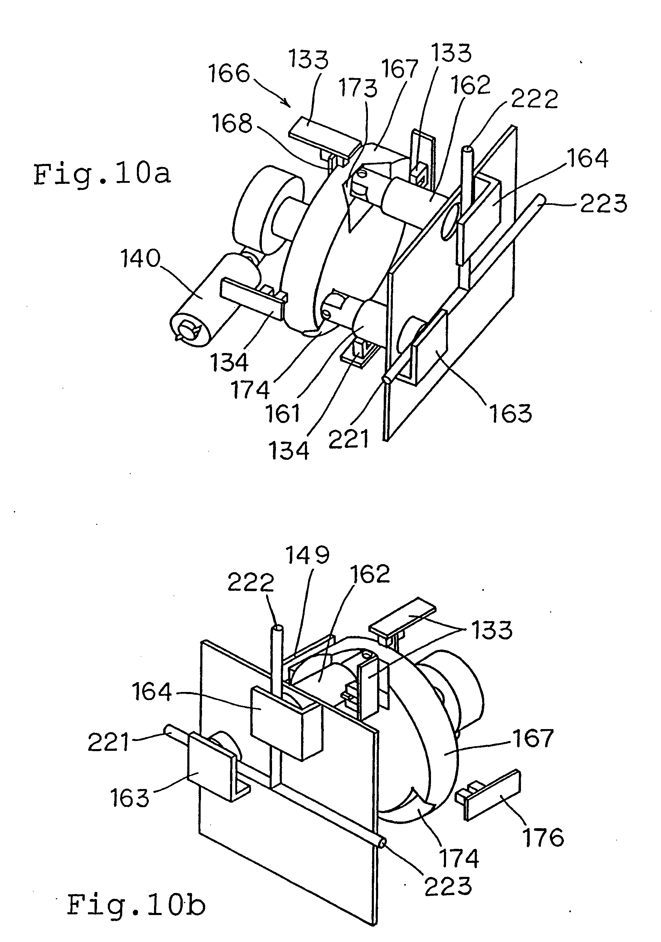

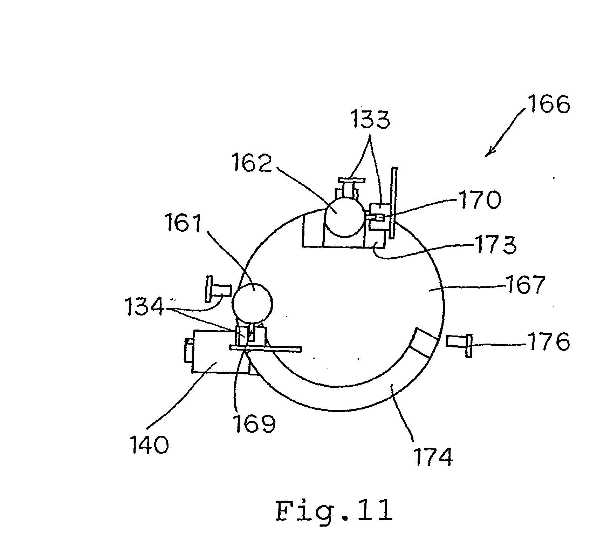

As a further alternative, as shown in Figs. 10a and 10b and Fig. 11, it is possible that first press member 161 and second press member 162 are supported slidably in the same direction to face first hold member 163 and second hold member 164, respectively, and to engage with a surface of rotatable cam member 167 of open or close interlock mechanism 166, on which concaves and convexes are formed.

As in open or close interlock mechanism 166, convex 168 of cam member 167 can be sensed by first block sensor 133 and second block sensor 134, and convexes 169 and 170 of first press member 161 and second press member 162 can be immediately sensed by first block sensor 133 and second block sensor 134.

In open or close interlock mechanism 166, since first and second press members 161, 162 release the blocking of first and second tubes 221, 222 by their trailing ends being fit in concave 173 of the surface of cam member 167. Thus, after one of the tubes is completely blocked, the blocking of the other can be released, thereby making it possible to reliably prevent a backflow of the contrast medium and the like.

In addition, since concave 174 in which both of first and second press members 161, 162 are simultaneously located is also formed on cam member 167 in open or close interlock mechanism 166, first and second tubes 221, 222 can be released from blocking simultaneously as desired, and first and second tubes 221, 222 can be easily loaded.

To ensure such operation, convexes 169, 170 of first press member 161 and second press member 162 are preferably sensed directly by first block sensor 133 and second block sensor 134 described above. However, it is possible to provide dedicated release sensor 176 for sensing both of first and second press members 161, 162 being fit in concave 174.

## Claims

1. A liquid injector for injecting a liquid into a patient from a syringe having a cylinder member and a piston member inserted slidably into the cylinder member, comprising:
a patient tube having a leading end connected to the patient;
a plurality of syringe tubes, each of their trailing ends being connected to each of a plurality of the syringes;
tube connecting means for connecting a trailing end of the patient tube to leading ends of a plurality of the syringe tubes;
a syringe hold member for removably holding a plurality of the syringes;
a plurality of syringe drive mechanisms, each of the mechanisms causing each of a plurality of the syringes to perform injection of the liquid by relatively moving the cylinder member and/or the piston member;
a plurality of tube block mechanisms, each of the mechanisms blocking each of a plurality of the syringe tubes to allow opening or closing thereof; and
interlock control means for interlocking operation of a plurality of the tube block mechanisms and a plurality of the syringe drive mechanisms to open only one of the syringe tubes and drive only one of the syringes associated with the opened syringe tube.

2. The liquid injector according to claim 1, wherein the number of each of the syringes, the syringe tubes, the syringe drive mechanisms, and the tube block mechanisms is two, and
the liquid injector further comprising an open or close interlock mechanism for interlocking opening or closing operation of the two tube block mechanisms such that one of them is opened when the other is closed.

3. The liquid injector according to claim 2, wherein a first one of the tube block mechanisms has a first press member disposed movably at a position for pressing a first one of the syringe tubes and a first hold member disposed opposite to the first press member through the first syringe tube,
a second one of the tube block mechanisms has a second press member disposed movably at a position for pressing a second one of the syringe tubes and a second hold member disposed opposite to the second press member through the second syringe tube, and
the open or close interlock mechanism has a press slider member supported slidably and having the first press member and the second press member formed thereon to be integral therewith.

4. The liquid injector according to claim 2, wherein a first one of the tube block mechanisms has a first press member disposed movably at a position for pressing a first one of the syringe tubes and a first hold member disposed opposite to the first press member through the first syringe tube,
a second one of the tube block mechanisms has a second press member disposed movably at a position for pressing a second one of the syringe tubes and a second hold member disposed opposite to the second press member through the second syringe tube, and
the open or close interlock mechanism has a press pivot member having the first press member and the second press member formed integrally and supported pivotally.

5. The liquid injector according to claim 2, wherein a first one of the tube block mechanisms has a first press member disposed movably at a position for pressing a first one of the syringe tubes and a first hold member disposed opposite to the first press member through the first syringe tube,
a second one of the tube block mechanisms has a second press member disposed movably at a position for pressing a second one of the syringe tubes and a second hold member disposed opposite to the second press member through the second syringe tube, and
the open or close interlock mechanism has a crank member supported pivotally on its own trailing end, a first link member for connecting a leading end of the crank member to the first press member, and a second link member for connecting the leading end of the crank member to the second press member.

6. The liquid injector according to claim 2, wherein a first one of the tube block mechanisms has a first press member disposed movably at a position for pressing a first one of the syringe tubes and a first hold member disposed opposite to the first press member through the first syringe tube,
a second one of the tube block mechanisms has a second press member disposed movably at a position for pressing a second one of the syringe tubes and a second hold member disposed opposite to the second press member through the second syringe tube, and
the open or close interlock mechanism has a cam member supported pivotally and having a concave and a convex with which the first press member and the second press member engage.

7. The liquid injector according to any one of claims 2 to 6, further comprising a first block sensor for sensing a first one of the syringe tubes being blocked and a second block sensor for sensing a second one of the syringe tubes being blocked,
wherein the interlock control means activates a second one of the syringe drive mechanisms after the first block sensor senses the blocking and activates a first one of the syringe drive mechanisms after the second block sensor senses the blocking.

8. The liquid injector according to any one of claims 1 to 7, further comprising a one-way valve for regulating the movement of the liquid in a direction from the syringe to the patient, the at least one one-way valve being provided for at least one of the patient tube and a plurality of the syringe tubes.
